# NEW EUROPEAN PATENT SPECIFICATION

(11) **EP 0 535 942 B2**
(45) Date of publication and mention of the opposition decision: **24.11.2004**
(45) Mention of the grant of the patent: 03.02.1999
(21) Application number: 92308930.4
(22) Date of filing: 30.09.1992
(51) Int. Cl.: C11D 3/50

(54) **Fabric treatment compositions comprising perfume particles**
Parfümteilchen enthaltende Textilbehandlungsmittel
Compositions pour le traitement de linge comprenantes des particules de parfum

(30) Priority: 02.10.1991 GB 9120952
(43) Date of publication of application: 07.04.1993
(73) Proprietor: UNILEVER PLC, London EC4P 4BQ (GB); UNILEVER N.V., 3013 AL Rotterdam (NL)
(72) Inventor: Garner-Gray, Peter F., Unilever Res. Port Sunl.Lab, Wirral, Merseyside L63 3JW (GB); Martin, Alexander, Unilever Res. Port Sunlight Lab, Wirral, Merseyside L63 3JW (GB); Martin, John R., Unilever Res. Port Sunlight Lab., Wirral, Merseyside L63 3JW (GB); Webb, Maurice, Unilever Res. Port Sunlight Lab., Wirral, Merseyside L63 3JW (GB)
(74) Representative: Tjon Tien Ril, Hon Kong Guno

(56) References cited:
- EP-A- 0 332 259
- EP-A- 0 332 260
- DD-A- 137 599
- DD-A- 248 508
- US-A- 4 536 315
- DATABASE WPI Section Ch, Week 7816, Derwent Publications Ltd., London, GB; Class D23, AN 78-29821A & JP-A-53 026 335 (SEIWA SANGYO KK) 11 March 1978

## Description

This invention relates to fabric treatment compositions containing perfume particles and in particular to laundry detergent or bleaching compositions, fabric softener compositions - especially dryer activated solid softener compositions. The particles allow the intensity of perfume in a composition to be controlled, suppress unwanted perfume loss and can act as a delivery means to the wash or rinse liquor or fabric.

Perfumes are used in compositions for two main reasons. The first is to disguise any chemical odours of the base ingredients and the second is to provide a pleasing odour to the article treated with the composition. Normally, one perfume performs both functions. Perfumes are often added directly to laundry compositions e.g. by spraying onto granular compositions. However, perfumes are, in general, volatile and many perfume ingredients can be lost from the product during processing or storage, or destroyed or damaged by contact with the highly alkaline conditions present in laundry compositions or by contact with some components of the composition (e.g. bleaches, enzymes). There is therefore a need for a carrier that will suppress unwanted perfume loss, or reduce degradation, or both.

A further disadvantage that arises from the direct addition of perfumes to compositions is that the same perfume must be used to perfume both the composition and the article to which it is delivered. There is no flexibility for example to allow a laundry composition to have one perfume and the cleaned fabric another perfume.

Another problem arises from the trend towards more concentrated products mainly for environmental reasons, for example high bulk density laundry detergent compositions, highly concentrated fabric softening compositions and solid deodorant sticks. These concentrated products are either used at lower dosage levels by the consumer or are diluted to make up the "normal" dose level. In order to ensure that these concentrated products give the same or a greater level of perfume delivery to fabric or liquor it is necessary to include the perfume at appropriately higher levels. This can lead to concentrated products having unacceptably heavy odours which are unattractive to the consumer. There is thus a need to control the intensity of the perfume in a composition, so that unacceptably heavy odours can be avoided.

In the past, attempts at solving the problem of unwanted loss or degradation of perfume have centred around the use of carriers impregnated with the perfume. For example EP 0 332 259A (Procter and Gamble/Sagel) discloses certain perfume particles formed by adsorbing a perfume onto silica. EP 0 332 260A (Procter and Gamble/Ladd) discloses the use of such particles in fabric softening compositions. In this prior art there is much emphasis on particle size, total pore volume and surface area of the silica since adsorption capacity is of prime importance. These alleged solutions, however, do not sufficiently overcome the problem of perfume loss, unacceptable intensity or inflexibility since the perfume is not captured by the carrier.

It is known from GB-A-1 570 608 (Doulton/Parkes) that ceramics of pore size less than 5 microns can be impregnated with a perfume to form an air freshener.

It is also know from JP-A-53/26335 (Seiwa Sangyo KK/Yasunaga) to absorb perfume on a granular powder which can be silica gel to form perfumed accessories such as necklaces or air fresheners. The one example discloses a perfumed Type B silica gel with a surface area of 350m²/g.

We have now found that the disadvantages of the prior art can be overcome, perfume intensity controlled and unwanted loss suppressed if the carrier has a minimum pore volume comprising pores of a certain size range.

Accordingly one aspect of the invention provides a fabric treatment composition containing surfactant or fabric softener and also comprising porous inorganic carrier particles which are silica having a perfume absorbed into said particles, characterised in that said particles have at least a pore volume of 0.2 ml/g consisting of pores with a diameter of 7 to 50A

The carrier particles are used to make perfumed compositions such as dry flowable detergent compositions, laundry bars and fabric softener compositions in solid, liquid or aerosol form. Use of the carrier particle can allow reduced levels of perfume to be added to the composition since unwanted loss is mitigated, can control the intensity of perfume in concentrated compositions so that both composition and treated article carry acceptable levels of perfume and can allow the composition and the article treated therewith to carry different perfumes. It is also possible that use of the perfumed carrier particle can allow either the composition or article treated therewith to be "unperfumed". The carrier particles when used in deodorant or antiperspirant compositions can have the advantage that triggered release provides a higher level of perfume when it is needed and avoids the practice of the user applying excess quantity of product.

While not wishing to be bound by theory it is thought that the advantageous effects of the invention are obtained due to local structuring of the perfume molecules at the carrier surface.

The perfume can be sprayed onto the inorganic carrier in various ways well known in the art. It can be added neat or with diluents, viscosity modifiers or wetting agents and may be absorbed at room temperature or at elevated temperatures or may be vacuum filled. As used herein the term "perfume" denotes one or a mixture of perfumed components, optionally mixed with a suitable solvent, diluent or carrier. Perfume components and mixtures thereof which can be used for the preparation of such perfumes may be natural products such as essential oils, absolutes, resinoids, resins or concretes, and synthetic perfume components such as hydrocarbons, alcohols, aldehydes, ketones, ethers, acids, esters, acetals, ketals or nitriles, including saturated and unsatured compounds, aliphatic, carbocyclic and heterocyclic compounds. Examples of such perfume components components are: geraniol, geranyl acetate, linalool, linalyl acetate, tetrahydrolinalool, citronellol, citronellyl acetate, dihydromyrcenol, dihydromyrcenyl acetate, tetrahydromyrcenol, terpineol, terpinyl acetate, nopol acetate, 2-phenylethanol, 2-phenylethyl acetate, benzyl alcohol, benzyl acetate, benzyl salicylate, benzyl benzoate, styrallyl acetate, amyl salicylate, dimethylbenzylcarbinol, trichloromethylphenycarbinyl acetate, p-tert.butylcyclohexyl acetate, isononyl acetate, vetiveryl acetate, vetiverol, alpha-n-amylcinnamic aldehyde, alpha-hexylcinnamic aldehyde, 2-methyl-3-(p-tert.butylphenyl)propanal, 2-methyl-3-(p-isopropylphenyl)propanal, 3-(p-tert.butylphenyl)propanal, tricyclodecenyl acetate, tricyclodecenyl propionate, 4-(4-hydroxy-4-methylpentyl)-3-cyclohexenecarbaldehyde, 4-(4-methyl-3-pentenyl)-3-cyclohexenecarbaldehyde, 4-acetoxy-3-pentyltetrahydropyran, methyl dihydrojasmonate, 2-n-heptylcyclopentanone, 3-methyl-2-pentyl-cyclopentanone, n-decanal, n-dodecanal, 9-decenol-1, phenoxyethyl isobutyrate, phenylacetaldehyde dimethyl acetal, phenylacetaldehyde diethyl acetal, geranonitrile, citronellonitrile, cedryl acetate, 3-isocamphylcyclohexanol, cedryl methyl ether, isolongifolanone, aubepine nitrile, aubepine, heliotropine, coumarin, eugenol, vanillin, diphenyl oxide, hydroxycitronellan, ionones, methyl ionones, isomethyl ionones, irones, cis-3-hexenol and esters thereof, indane musk fragrances, tetralin musk fragrances, isochroman musk fragrances, macrocyclic ketones, macrolactine musk fragrances, ethylene brassylate, aromatic nitromusk fragrances.

Suitable solvents, diluents or carriers for perfumes as mentioned above are for example: ethanol, isopropanol, diethylene glycol monoethyl ether, dipropylene glycol, diethyl phthalate and triethyl citrate.

For certain applications, the perfume compositions are substantive to maximise the effect on fabric. A substantive fragrance is one which contains a sufficient percentage of substantive fragrance materials that when the fragrance is used at normal levels it deposits a desired odour on the fabric. The perfumes may also be deodorant perfumes, anti-microbial perfumes or have other functionalities desirable for certain applications. Examples are disclosed in GB-A-1 566 538, GB-A-1 572 949, EP-A-62368, EP-A-49543 EP 3171, EP-A-1 471 191, EP-A-430 315 and EP-A-91200560 (all in the name of Unilever).

For some applications the perfume particles preferably release perfume when they are wetted e.g. with an aqueous fluid, such as a wash liquor. When the particles are attached to fabrics they can be activated by wetting with an undesirable fluid such as sweat or urine which triggers release of the perfume. The perfume can either mask or otherwise suppress unwanted odours or signal that other action is required. The particles can be applied to fabrics either from a wash, rinse liquor or in the dryer or by direct application from a laundry bar

Inorganic carriers for use in the present invention are certain silicas all with pore volume of at least 0.2ml/g consisting of pores with a diameter between 7 and 50Å.

Between 0.2ml/g and 1.5ml/g may consist of pores with diameter of between 7 and 50 Å. The inorganic carrier may have a pore volume of at least 0.lml/g consisting of pores with diameters of between 11 and 40Å.

In the context of the present invention particles sizes above 100 microns are determined by sieving, particle sizes below 100 microns are determined by a Malvern 3600 particle analyser.

The particles of the present invention can also be formed into aggregates of two or more particles to make aggregates of several particle diameters, for example 1000µm.

Although the inorganic carrier has a pore volume of preferably at least 0.2ml/g to 1.5ml/g consisting of pores with a diameter of between 7 and 50Å, the total pore volume of the carrier can be greater and include pores with a diameter greater than 50Å. For example the total pore volume can be between 0.2ml/g and 2.5ml/g.

In the context of the present invention the porosity characteristics of the carrier are determined by nitrogen absorption isotherm. The volume, Vₐ, of nitrogen absorbed in pores with diameters between 17Å and 50Å is determined according to the method of Barrett, Joyner and Halenda, "*JACS*", 73, 373, (1951) from the absorption data. The volume, V_{b}, of nitrogen absorbed in pores of between 7Å and 20Å in diameter is determined using T-plot analysis according to the method of Lippons and deBoer, "*J Catalysis",* 4, 319, (1965). V_{b} is calculated from the intercept at t=0 of a line fitted to the linear portion of the t-plot curve within the range , t=3 to t=16A. If, within this range, there are two linear regions, the line with the lower gradient is used. If there are three linear regions the line is fitted to the one giving the lowest intercept at t=0. Inorganic carriers suitable for use in the present invention have a volume of Vₐ plus V_{b} of at least 0.2ml/g.

Inorganic carriers suitable for use in the present invention include silicas such as Gasil 200 also referred to herein as GASIL ex Crosfield Chemicals with a volume Vₐ + V_{b} of 0.64ml/g, an average particle size of 10-15 microns and a surface area of 730m²/g; Sorbsil ex Crosfield Chemicals with a volume Vₐ + V_{b} of 0.69ml/g, average particle size of 50-250 microns, and surface area of 730m²/g; Sorbsil C30 ex Crosfield Chem. with a volume of Vₐ + V_{b} of 0.98ml/g particle size of 60 microns, and surface area of 640m²/g and MD 263 a silica as described in Example 3 of EP-A-287 232 with a volume Va + Vb of 0.28ml/g, a surface area of 730m²/g and a particle size of 25-30 microns. The inorganic carriers suitable for use herein are preferably not hydrophobic as for example described in our copending application GB9120951.0 subsequently published as EP-A-536942.

The perfumes absorbed into the carrier are preferably added at levels below the theoretical maximum absorption capacity of the carrier. For an "unperfumed" composition or low perfume intensity composition the perfume absorbed into the carrier is added at levels below the absorption capacity of the pore volume consisting of pores having a pore diameter of 7 to 50Å. For a composition that is perfumed differently from the article treated therewith, the perfume intended to be delivered to the article is first absorbed into the carrier at a level below the absorption capacity of the pore volume consisting of pores having a pore diameter of 7 to 50Å. The perfume intended for the composition may then be either absorbed by the carrier up to the level of its maximum capacity or by the composition. Preferably the ratio by weight of carrier to perfume is less than 25:1, more preferably between 12:1 and 3:1 e.g. 10:1 or 6:1. The level of addition of perfume should be chosen to give free flowing particles.

One preferred aspect of the invention relates to the use of the perfumed inorganic carrier particles mentioned hereinabove in detergent compositions. Such compositions typically comprise detersive surfactants and/or detergency builders, bleaches and, optionally, additional ingredients such as enzymes, fabric brighteners, free perfume, fabric softeners e.g. clay or cationic softeners.

Accordingly a preferred aspect of the invention provides a detergent composition comprising at least one surfactant and from 0.1% to 60% by weight of a porous inorganic carrier particle having a pore volume of at least 0.2ml/g consisting of pores with a diameter of 7 to 50Å and having a perfume absorbed into said particle.

Surfactants useful in the detergent compositions herein include well-known anionic, nonionic, amphoteric and zwitterionic surfactants. Typical of these are the alkyl benzene sulphonates, alkyl sulphonates, allyl- and alkylether sulphates, primary alkyl sulphates, alkoxylated alcohols, alpha-sulphonates of fatty acids and of fatty acid esters, alkyl betaines or polyalkyl glycosides all known in the detergent art. The surfactant is preferably present at a level from 5% to 60% by weight, more preferably 10% to 50%.

Useful detergency builders for the detergent compositions herein include any of the conventional inorganic and organic water soluble builder salts as well as the various insoluble salts. Examples are alkali metal carbonates, borates, phosphates, polyphosphates, tripolyphosphates, bicarbonates, silicates, sulphates, calcite seeded carbonates, zeolites such as zeolite 4A and citric acid salts. The builder is preferably present at a level between 5 and 80% by weight, more preferably 10 and 60% by weight.

Useful bleaches include halogen bleaches, peroxyacids such as diperoxydodecanedioic acid or phthaloylaminoperoxycaproic acid, or bleach systems that comprise a peroxide compound which liberates hydrogen peroxide in aqueous solution and an activator. Hydrogen peroxide sources are well known in the art, and include compounds such as alkali metal perborates, percarbonates, persulphates and persilicates, and urea peroxide and the like; particularly percarbonate and perborate.

Activators such as are described in GB-A-836,988, GB-A-855,735, GB-A-907,356, GB-A-907,358, GB-A-970,950, GB-A-1,003,310, GB-A-1,246,339, US-A-3,332,882, US-A-4,128,494, CA-A-844,481, and ZA-A-68/6344 are preferred. Particularly preferred are N,N,N,N-Tetraacetylethylenediamine (TAED), 1,2,3,4,6-Pentaacetylglucose (GPA), Sodium-p-acetoxybenzenesulphonate (SABS), Sodium-p-benzoyloxybenzenesulphonate (SBOBS), Sodium-p-nonanoyloxybenzenesulphonate (SNOBS), Sodium-p-3,5,5-trimethylhexanoyloxy-benzenesulphonate (iso-SNOBS), 2-N,N,N-Trimethylammonioethyl-4-sulphophenylcarbonate (CSPC), 2-N,N,N-Trimethylammoniopropionitrile tosylate (TAP), and transition metal catalysts.

As optional ingredients the detergent compositions can comprise additional perfume carriers.

Another preferred aspect of the invention relates to the use of the perfumed inorganic carrier particles mentioned hereinabove in fabric softener compositions. Such fabric softener compositions are particularly those which are attached to substrates for use in laundry dryers.

Accordingly a preferred aspect of the invention provides a dryer activated fabric softener composition comprising a fabric softener and porous inorganic carrier particle having a pore volume of at least 0.2ml/g consisting of pores with a diameter of 7 to 50Å and having a perfume absorbed into said particle.

The fabric softener is typically cationic and usually a quaternary ammonium salt of formula [R₁R₂R₃R₄N⁺]Y⁻ wherein one or two of the R groups is an aliphatic radical or an alkylphenyl or alkylbenzyl radical having from 10 to 20 carbon atoms in the chain. The remaining R groups being selected from C₁-C₄ alkyl and C₂-C₄ hydroxyalkyl. Preferably the hydrophobic R groups are attached to the N atom via one or more ester links. The quaternary ammonium compounds useful herein also comprise imidazolinium compounds. Y is typically a halide, nitrate, bisulphate, ethylsulphate or methyl sulphate. The fabric softener may also be an amine.

The fabric softening compositions of the invention preferably comprise from 5% to 80% by weight of fabric softener, more preferably from 5% to 50%. The compositions also preferably comprise from 1% to 60% of the perfumed inorganic carrier particles. Use of the carrier in fabric softening compositions is particularly advantageous for concentrated compositions which require high perfume concentrations.

The fabric softening compositions may also contain various optional ingredients including auxiliary softeners, soil release agents, free perfume or brighteners.

The following non-limiting examples illustrate the compositions of the present invention.

### Example 1

The perfumed inorganic carrier particles hereinafter described were made on a laboratory scale by adding the appropriate amount of perfume dropwise, with stirring to a jar containing the carrier. The jar was then sealed and allowed to equilibriate overnight.

| | Particle | | | | | | |
|---|---|---|---|---|---|---|---|
| Ingredient (% by weight) | B | C | D | E | F | G | H |
| Perfume¹ | 14.3 | 16.7 | 20 | 14.3 | 14.3 | 20 | 14.3 |
| Silica-Gasil 200 | 85.7 | 83.3 | 80 | - | - | - | - |
| Silica-Sorbsil | - | - | - | 85.7 | - | - | - |
| Silica-MD263PS | - | - | - | - | 85.7 | 80 | - |
| Sorbsil C30 | - | - | - | - | - | - | 85.7 |

| | | | | | | | |
|---|---|---|---|---|---|---|---|
| 1 The perfume used was amyl acetate. | | | | | | | |

### Example 2

The extent to which the perfumed inorganic carrier particles supress the loss of perfume from the carrier was assessed by measuring the equilibrium vapour pressure of perfume above a given particle compared to that above free perfume. This then allows a calculation of the percentage reduction in vapour pressure and thus allows a quantitative comparison between different carriers.

The apparatus used comprised a two-necked, 500ml flask in a thermostated water bath. One neck of the flask was connected via a short flexible connection to a water manometer (glass, 2mm internal bore). The other neck was fitted with a dropping funnel to allow samples to be placed in the flask. The system was stabilised at 65°C with the tap of the dropping funnel in the open position. The vapour pressure above a free perfume sample was measured by introducing the perfume via the funnel, closing the tap and noting the manometer reading once a steady state has been reached. For particulate samples the same procedure was followed except that the solid was introduced using a filter funnel which was then immediately replaced with the dropping funnel to seal the flask.

| Particle | Volume of pores with Pore Diameter between 7 - 50Å (Vₐ + V_{b}) | Carrier Perfume Ratio | % Reduction Vapour Pressure |
|---|---|---|---|
| B | 0.64 | 6:1 | 90 |
| C | 0.64 | 5:1 | 90 |
| D | 0.64 | 4:1 | 80 |
| E | 0.69 | 6:1 | 90 |
| F | 0.28 | 6:1 | 86 |
| G | 0.28 | 4:1 | 83 |
| H | 0.98 | 6:1 | 90 |
| Silica-MicrosiIGP¹ | 0.04 | 4:1 | 17 |
| | 0.04 | 6:1 | 32 |
| Silica- Cab-0-Sil M5² | 0.07 | 6:1 | 28 |
| | 0.07 | 5:1 | 14 |
| Zeolite 4A³ | 0 | 6:1 | 14 |
| Type B silica⁴ | 0.05 | 6:1 | 73 |
| Type B silica⁴ | 0.05 | 4:1 | 52 |
| EP 10X⁵ | 0.02 | 6:1 | 45 |
| EP 10X⁵ | 0.02 | 4:1 | 23 |

| | | | |
|---|---|---|---|
| 1 Microsil GP is a precipitated silica ex Crosfield Chemicals | | | |
| 2 Cab-o-sil M5 is a fumed silica ex Cabot Corporation | | | |
| 3 Zeolite 4A is a zeolite ex Crosfield Chemicals | | | |
| 4 Type B silica is CC1, Type B silica gel with a surface area of 340m²/g ex Crosfield Chemicals | | | |
| 5. EP 10X is a silica gel ex Crosfield Chemicals | | | |

Particles A to H are as in Example 1.

These results show that perfumed inorganic carrier particles according to the invention (A to H) are more effective in suppressing perfume loss from the carrier than those carriers with pore volumes outside the claimed range.

### Example 3

The extent of perfume loss suppression by the particles in a detergent composition was assessed by equilibrium vapour pressure measurements. All measurements were at 37°C.

| | Composition (g) | | |
|---|---|---|---|
| | I | II | III |
| New System Persil Automatic¹ | 4 | 4 | 4 |
| Amyl acetate² | 0.3 | - | - |
| Particle B³ | - | 2.1 | - |
| % Vapour Pressure (cm H₂O) reduction (relative to free amylacetate) | 20 | 78 | 83 |

| | | | |
|---|---|---|---|
| 1. A commercially available detergent ex Lever Bros. dried at 100°C overnight. | | | |
| 2. Sprayed onto powder after drying. | | | |
| 3. From Example 1. 2.1g of Particle B comprises 0.3g of amyl acetate. | | | |

These results show that carrier particles according to the invention suppress perfume loss from detergent compositions.

### Example 4

The effect of water on the inorganic carrier was assessed by treating 30-35 mg of various carriers with perfume and sealing in glass jars. Six panellists were then provided with two samples of each carrier, to one of which samples 0.2 mls of water had been added. Both samples were then left for 5 minutes to reach equilibrium. Perfume intensity scores were then allocated for each sample. The mean scores for each sample wet and dry were evaluated and the dry score subtracted from the wet one. The difference between these figures provides a measure of the perfume release by the water trigger.

| Sample | Va + Vb (7-50Å) | Perfume Intensity Wet - Dry |
|---|---|---|
| I Gasil 200 | 0.64 | 35 |
| J Sorbsil P80 | 0.70 | 17 |
| K Sorbsil C30 - 10P | 0.79 | 5.5 |
| L Sorbsil C60 - 10P | 0.25 | 2.5 |
| M Sorbsil C500 - 15/20 | 0.01 | 0 |
| (Gasil 200 and all Sorbsils are silicas ex Crosfield Chemicals). | | |

These results show that perfumed inorganic carrier particles according to the invention give particularly effective perfume release when exposed to an aqueous trigger.

### Example 5

The effect of water on perfumed inorganic carrier particles held for various periods of time at elevated temperature was assessed by soaking 45g of silica (Sorbsil P80 ex Crosfield as used in Example 4) in a solution of 1 g of perfume in absolute alcohol. The liquid was then poured into a large petri dish and allowed to dry under ambient conditions to a fine powder.

The carrier was then placed in a fan over and triggered release measured as in Example 4 after various times.

| Time in oven at 40°C (hrs) | Perfume Intensity Wet - Dry | |
|---|---|---|
| | Eugenol | Citronellol |
| 0 | 29 | 28 |
| 1 | 25 | 23 |
| 2 | 28 | 22 |

These results show that carrier particles according to the invention maintain their ability to release perfume in response to a trigger after several hours exposure to body heat.

### Example 6

The effect of inorganic carrier to perfume ratio was studied by measuring the equilibrium vapour pressure of perfume over particles at varying carrier to perfume ratios. All particles contained 0.3g of amylacetate ex Aldrich, measurements were at 65°C.

| Carrier/Perfume Ratio | Vapour Pressure over Gasil 200 (cm H₂O) | Vapour Pressure over MD263PS |
|---|---|---|
| 6:1 | 4.0 | 5.7 |
| 5:1 | 3.6 | - |
| 4:1 | 8.0 | 6.9 |
| 3:1 | 19.8 | 12.2 |
| Free perfume | 40.0 | 40.0 |

The data shows that the Vapour pressure rises as the micropores tend to saturation.

### Example 7

The effectiveness of hydrophilic silica for delivering perfume from standard and concentrated washing powders was assessed. 100g samples of detergent powders were assessed by panel testing (20 people) and perfume strengths scored by Magnitude Estimation. Standard powders (Persil) contained 0.2% LP927LL and powder concentrates (Radion Micro) contained 0.5% LP927LL perfume. The reference samples simply had perfume added to the powders and the samples thoroughly mixed before use. With the carrier systems, the perfume was mixed thoroughly into the porous solid and the mix allowed to stand overnight before being dispersed into the detergent powder. Perfume intensities were assessed soon after sample preparation.

| Powder | Carrier | Carrier/Perf. Ratio | Perf. Intensity Score | | | |
|---|---|---|---|---|---|---|
| Standard | none (ref) | - | 118 | | | |
| " | Sorbsil | 6:1 | 50 | | | |
| Concentrate | none (ref) | - | | 149 | 126 | 120 |
| " | Gasil | 6:1 | | 95 | | |
| " | Sorbsil | 6:1 | | 79 | | |
| " | Gasil | 4.5:1 | | | 96 | |
| " | Sorbsil | 4:5:1 | | | 63 | |
| " | Gasil | 3.5:1 | | | | 99 |
| " | Sorbsil | 3:5:1 | | | | 68 |

The examples show much lower perfume intensities for the powders containing microporous carriers at carrier/perfume ratios from 6:1 to 3.5:1.

### Example 8

In this test, simulated wash liquors were prepared by adding 2g standard "Persil" powder (freshly prepared) to 500ml tap water at 40°C, the powders dispersed, that liquor cooled rapidly to 20°C and perfume strengths assessed by Magnitude Estimation within an hour of sample preparation.

| Powder | Carrier | Carrier/Perfume Ratio | Perfume Intensity Score | | |
|---|---|---|---|---|---|
| Standard | none (ref) | - | 119 | 92 | 83 |
| " | Gasil | 6:1 | 104 | 88 | |
| " | Sorbsil | 6:1 | | | 76 |
| " | EP10X (large pore) | 6:1 | | | 84 |
| " (unperfumed) | | - | 28 | | |

The results indicate that perfume intensities in wash liquors containing hydrophilic carriers are virtually the same as the reference samples shortly after their preparation, i.e. that their perfume deliveries to wash liquors are high.

The example was repeated with a powder concentrate. In this test, 1.33g of detergent powder concentrate (Radion Micro, freshly prepared with perfume or perfume/carrier) was weighed into a 1 litre glass jar, 500ml tap water at 20°C added, the jar sealed and the mixture shaken at intervals over the next 10/15 minutes. During the subsequent 15 minutes, a small panel (10 people) was then asked to rank the perfume strengths over the liquors.

| Sample | Perf.Strength Rel.to Ref.(no carrier) | | |
|---|---|---|---|
| | Weaker | Same | Stronger |
| hydrophilic Gasil | 1 | 2 | 7 |
| hydrophilic Sorbsil | 6 | 1 | 3 |

Comments associated with these results indicate that the hydrophilic silicas give wash liquor perfume strengths which are similar to that of the directly perfumed powder. This shows that the perfume can be delivered effectively from the silica carrier.

### Example 9

The effectiveness of hydrophilic perfume carriers according to the invention was assessed in a number of various washing powder formulations.

Powders employed :-
A) high anionic powder -
   17.6% LAS, 4.3% soap, 2.9% nonionic (STP/carbonate)
B) high nonionic powder -
   22.2% nonionic
   (zeolite/carbonate)
C) high nonionic powder :-
   2.8% PAS, 25.16% nonionic
   (zeolite)
D) high nonionic powder :-
   1.9% PAS, 17.5% nonionic
   (zeolite/carbonate/bleach)
E) Radion Micro (as reference) -
   6.26% LAS, 1.7% soap, 9.77% nonionic
   (zeolite/carbonate/bleach)

Perfume intensities were assessed soon after sample preparation. The assessment was by panel testing - panels of about 20 people scoring the perfume strength on a 0-13 point scale.

Hydrophilic Sorbsil silica carriers were used. Each had the perfume LP927LL at ratio of 6:1 (silica/perfume) and with the perfume level in all samples at 0.6%. The mean perfume intensity scores are shown below,

| Powder | A | B | C | D | E |
|---|---|---|---|---|---|
| without carrier | 8.14 | 6.87 | 7.63 | 7.57 | 7.70 |
| with Sorbsil carrier | 2.38 | 5.50 | 5.22 | 4.28 | 6.04 |

### Example 10

Hydrophilic silicas do not generally give moderate to large perfume deliveries to washed fabric but they are capable of delivering small increases in perceived perfume levels.

Whilst Laboratory tests involving a standard wash/rinse (line dry) treatment of Terry cotton failed to find any significant increases in perfume level on dry fabric. On re-moistening, a reproducable increase in perfume score with large particle silicas but not with small particle silicas was observed, see table below. The standard treatment consists of taking 0.056g of the inorganic carrier particle perfumed at a 6:1 ratio with LP927 LL ex Quest International Limited and mixing it with 4g of New System Persil Automatic ex Lever. The mixture is then added to 1 litre of demineralised water at 35°C. Terry cotton swatches are then added to the liquor in a tergotometer to give a liquor to cloth ratio of 20: 1. The swatches are washed at a temperature of 45°C for 15 minutes and rinsed for 5 minutes in cold water. The cloths are then wrung out by hand and line dried. A perfume intensity score is determined for each cloth by a method of panel assessment.

| Perfume Score | | | | | |
|---|---|---|---|---|---|
| Control | | Gasil | | Sorbsil | |
| Dry | Moist | Dry | Moist | Dry | Moist |
| 0.47 | 0.45 | 0.57 | 0.45 | | |
| 0.87 | 0.53 | 0.69 | 0.64 | | |
| 0.51 | 0.30 | | | 0.44 | 0.66 |
| 0.33 | 0.38 | | | 0.40 | 0.71 |
| 0.36 | 0.48 | | | 0.40 | 0.79 |
| 0.65 | 0.46 | | | 0.63 | 0.90 |

This would seem to suggest that silica/perfume deliveries from both silicas are very small such that perfume levels are not noticed on the dry fabric but that, with the larger particle silica, there is sufficient material present to give a moisture triggered boost to the perceived perfume strength.

From this it is concluded that large particle hydrophilic silicas can deliver low, but noticeably higher levels of perfume to washed fabric than control powders, although a moisture trigger may be needed. Small particle hydrophilic carriers give inferior delivery properties.

### Example 11

This example shows the delivery of perfume from a nonaqueous liquid detergent.

The liquid was unperfumed and comprised:
50% nonionic
6% ABS
17% Na carbonate
6% Ca carbonate
10.5% Na perborate plus minor components to 100% perfume level would be 0.5%

Perfume (LP927LL) (with and without silica carriers) was incorporated into the above at 0.5%.

100ml samples were prepared and used to gauge perfume intensity over the liquids (panel test) on a 0-13 point scale. Samples were removed for use in our perfume delivery test (standard Tergotometer wash/rinse/line dry regime : wash liquor conc. was 2.4g/L). The results detailed below are from freshly prepared samples.

### PERFUME DELIVERY TO TERRY COTTON.

Dry cloths were assessed for perfume intensity on a 0-5 point scale.

| Silica Carrier | Mean Fabric Score |
|---|---|
| hydrophilic Sorbsil | 0.80 |
| hydrophilic Gasil | 0.32 |
| NONE (control) | 0.18 |

### Example 12

Tumble dryer sheets were prepared by incorporating 56g of Gasil 200/DAC 8987 perfume (ex. Quest)(6:1 silica/perfume ratio) into a melt of 192g Varisoft CS 132u fabric conditioner and coating a support with the mixture. When cold the sheets weighed 2-2.5g. Similar reference sheets were prepared by coating the support with a melted mixture of 192g Varisoft CS 132u/8g perfume. In both cases the sheets were satisfactory in appearance, feel and perfume strength. Washed polycotton (1Kg) was tumble dried in a Whirlpool drier with one of the above sheets. The dried fabric was assessed for perfume strength by a panel of 20 people.

| Tumble Drier Sheet | Mean Score |
|---|---|
| Gasil 200 sheet | 1.48 |
| Reference sheet | 1.27 |

The example shows that satisfactory sheets can be prepared with the perfumed particles and that perufme delivery from the test sheets is better than from the control.

Gasil, Sorbsil, Acumist, Microsil, Cab-O-Sil,Varisoft and Persil used in the above text are trademarks.

## Claims

1. A fabric treatment composition containing surfactant or fabric softener and also comprising porous inorganic carrier particles which are silica having a perfume absorbed into said particles, **characterised in that** said particles have at least a pore volume of 0.2 ml/g consisting of pores with a diameter of 7 to 50Å.

2. A composition as claimed in claim 1 wherein the particle has at least a pore volume of 0.1 ml/g consisting of pores with a diameter of 11 to 40Å.

3. A composition as claimed in any preceding claim wherein the particle has a carrier to perfume ratio by weight of less than 25:1.

4. A detergent composition according to any one of claims 1 to 3 comprising at least one surfactant and from 0.1% to 60% by weight of the porous inorganic carrier particles.

5. A fabric softener composition according to any one of claims 1 to 3 comprising a fabric softener and from 0.1 % to 60% by weight of the porous inorganic carrier particles.

## Patentansprüche

1. Ein Textilbehandlungsmittel, enthaltend Tensid oder Gewebeweichmacher und ebenfalls umfassend poröse anorganische Trägerteilchen, welche Kieselsäure sind, mit einem in die erwähnten Teilchen absorbierten Parfüm, **dadurch gekennzeichnet, daß** die erwähnten Teilchen ein Porenvolumen von zumindest 0,2 ml/g, bestehend aus Poren, mit einem Durchmesser von 7 bis 50 Å, haben.

2. Ein Mittel, wie in Anspruch 1 beansprucht, worin das Teilchen ein Porenvolumen von zumindest 0,1 ml/g aufweist, bestehend aus Poren mit einem Durchmesser von 11 bis 40 Å.

3. Ein Mittel, wie in irgendeinem der vorstehenden Ansprüche beansprucht, worin das Teilchen ein Träger-zu-Parfüm-Gewichtsverhältnis von weniger als 25 : 1 hat.

4. Eine Waschmittelzusammensetzung gemäß irgendeinem der Ansprüche 1 bis 3, enthaltend zumindest ein Tensid und von 0,1 bis 60 Gewichtsprozent der porösen anorganischen Trägerteilchen.

5. Ein Gewebeweichmacher-Mittel gemäß irgendeinem der Ansprüche 1 bis 3, enthaltend einen Gewebeweichmacher und 0,1 bis 60 Gewichtsprozent der porösen anorganischen Trägerteilchen.

## Revendications

1. Composition de traitement des textiles contenant un tensioactif ou un adoucissant des textiles et comprenant également des particules de support minérales poreuses qui sont la silice ayant un parfum absorbé dans lesdites particules, **caractérisée en ce que** lesdites particules ont au moins un volume de pores de 0,2 mL/g consistant en pores d'un diamètre de 7 à 50 angströms.

2. Composition selon la revendication 1, dans laquelle la particule a au moins un volume de pores de 0,1 mL/g consistant en pores d'un diamètre de 11 à 40 angströms.

3. Composition selon l'une quelconque des revendications précédentes, dans laquelle la particule a un rapport pondéral support/parfum de moins de 25:1.

4. Composition détergente selon l'une quelconque des revendications 1 à 3, comprenant au moins un tensioactif et de 0,1 à 60 % en poids des particules de support minérales poreuses.

5. Composition d'adoucissant des textiles selon l'une quelconque des revendications 1 à 3, comprenant un adoucissant des textiles et de 0,1 à 60 % en poids de particules de support minérales poreuses.
